# EUROPEAN PATENT SPECIFICATION

(11) **EP 3 834 822 B1**
(45) Date of publication and mention of the grant of the patent: **25.10.2023**
(21) Application number: 19846017.2
(22) Date of filing: 07.08.2019
(51) Int. Cl.: A61K 9/51, A61K 35/76, C12N 15/861, A61K 9/107, A61K 47/34

(54) **POLYMER NANOPARTICLE COMPOSITION FOR DELIVERING VIRUS, AND PREPARATION METHOD THEREFOR**
POLYMERNANOPARTIKELZUSAMMENSETZUNG ZUR ABGABE VON VIREN UND VERFAHREN ZU IHRER HERSTELLUNG
COMPOSITION DE NANOPARTICULES POLYMÈRES POUR L'ADMINISTRATION DE VIRUS, ET SON PROCÉDÉ DE PRÉPARATION

(30) Priority: 07.08.2018 KR 20180092089
(43) Date of publication of application: 16.06.2021
(73) Proprietor: Samyang Holdings Corporation, Seoul 03129 (KR)
(72) Inventor: CHOI, Joung Woo, Seoul 06506 (KR); KIM, Sang Hoon, Suwon-si Gyeonggi-do 16509 (KR); NAM, Hye Yeong, Seongnam-si Gyeonggi-do 13557 (KR); CHO, He Len, Seongnam-si Gyeonggi-do 13457 (KR); YUN, Min Hyuk, Yongin-si Gyeonggi-do 16899 (KR); KIM, Goo Young, Yongin-si Gyeonggi-do 16872 (KR); LEE, So Jin, Seoul 02468 (KR)
(74) Representative: Schön, Christoph
(86) International application number: PCT/KR2019/009893
(87) International publication number: WO 2020/032581

(56) References cited:
- WO-A1-2005/107813
- WO-A1-2018/085658
- JP-A- 2016 520 567
- KR-A- 20070 002 063
- KR-A- 20130 069 197
- KR-A- 20170 073 528
- XIA D. ET AL.: "Microencapsulation of recombinant adenovirus within poly-DL-lactide-poly(ethylene glycol) microspheres for enhanced gene transfection efficiency and inhibitory effects on hepatocellular carcinoma cells in vitro", MOLECULAR MEDICINE REPORTS, vol. 12, no. 2, 1 April 2015 (2015-04-01), pages 2336-2342, XP055685470, GR ISSN: 1791-2997, DOI: 10.3892/mmr.2015.3578
- DWIVEDI V. ET AL.: "Biodegradable Nanoparticle-Entrapped Vaccine Induces Cross-Protective Immune Response against a Virulent Heterologous Respiratory Viral Infection in Pigs", PLOS ONE, vol. 7, no. 12, 11 December 2012 (2012-12-11), page e51794, XP055223176, DOI: 10.1371/journal.pone.0051794
- XIA, D. et al.: "Microencapsulation of recombinant adenovirus within poly-DL- lactide-poly(ethylene glycol) microspheres for enhanced gene transfection efficiency and inhibitory effects on hepatocellular carcinoma cells in vitro", Molecular Medicine Reports, 2015, pages 2336-2342, XP055685470,

## Description

### TECHNICAL FIELD

The present invention relates to a virus-containing composition which comprises a virus for treating or preventing disease as an active ingredient, and a preparation method thereof.

### BACKGROUND ART

Vectors are commonly used as means for efficient delivery of genes for treatment into human cells. Vectors are divided into viral vectors and non-viral vectors.

Since viral vectors are expressed well in human cells and have advantages of good penetration and adhesion, they are widely used by biodrug manufacturers but with a potential risk in safety. Representative viral vectors are retrovirus and adenovirus, and in order to efficiently utilize them in treatment of cancers or various incurable diseases, the vector is modified or a chimera virus is developed mainly based on adenovirus. Modification of vector can include modification of the protein of the virus itself or incorporation of immunomodulatory into the vector. However, in case of intravenous administration, viral vectors cause hepatotoxicity due to accumulation in liver, and furthermore they are rapidly removed from blood, resulting in low delivery rate to tumor, and thus they are mainly used for topical administration only.

In contrast, non-viral vectors-although they are less efficient than viral vectors-have advantages of less side-effect in terms of in vivo safety and low production cost in terms of economy. The most representative ones among non-viral vectors are a complex of cationic lipid and nucleic acid (lipoplex) and a complex of polycationic polymer and nucleic acid (polyplex). Such a cationic lipid or polycationic polymer stabilizes nucleic acid by forming a complex through electrostatic interaction with the nucleic acid and increases intracellular delivery, and for these reasons, various researches thereof have been conducted. However, the results showed that such non-viral vectors were not appropriate for use as drug since they caused serious toxicity-although they are less toxic than viral vectors-in case of use in an amount necessary to obtain sufficient effect.

At present, hybrid vectors have been developed by combining the advantages of virus and non-virus ingredients. A strategy suggested for overcoming the limitation of CAR-dependency and immunogenicity of adenovirus is modification with a polymer which can pass through the surface of adenovirus without need of CAR-mediated endocytosis. Modification of adenovirus with cationic polymer or lipid strengthens virus-mediated gene delivery. However, such strategies do not draw targeted tumor-specific, virus-mediated gene delivery because the injected polymer/lipid-modified viruses are rapidly transferred to the non-targeted peripheral tissues. In addition, virus drugs are characterized in that, when they are introduced into body by using cationic non-viral vector, the delivery efficiency to tumor reduces remarkably. This is because the virus cannot go to the desired tissue due to non-specific binding with cationic polymer. Thus, it is necessary to develop a formulation for strengthening the deliverability and stability of virus, and a preparation method thereof.

Meanwhile, in order to provide a mixed polymer nanoparticle composition which solubilizes a large amount of poorly soluble drug and has good stability in aqueous solution, Korean Laid-open Patent Publication No. 10-2003-0032897 discloses a mixed polymer nanoparticle composition comprising an amphiphilic block copolymer consisting of a hydrophilic block and a hydrophobic block, and a polylactic acid derivative having carboxylic acid terminal group, which can form polymer nanoparticles in body fluid or aqueous solution; and a pharmaceutical composition comprising a poorly soluble drug which is contained within polymer nanoparticle formed from the mixed polymer nanoparticle composition.

### CONTENTS OF THE INVENTION

### PROBLEMS TO BE SOLVED

As a result of efforts exerted to increase delivery efficiency of virus, for example, adenovirus as oncolytic virus, the present inventors have confirmed that in case of entrapping adenovirus in polymer nanoparticle by mixing of wild-type adenovirus with amphiphilic block copolymer and salt of polylactic acid dissolved in organic solvent and emulsification under a monophase system for complex formation, the stability, safety and expression efficiency in targeted living tissue of adenovirus can be increased, and thus have completed the present invention.

Accordingly, the purpose of the present invention is to provide a composition which can effectively deliver virus into body.

Another purpose of the present invention is to provide a method for preparing a composition which can effectively deliver virus into body.

### TECHNICAL MEANS

A composition according to the present invention is defined in appended claim 1.

In addition, a method for preparing a composition according to the present invention is defined in appended claim 10.

### EFFECT OF THE INVENTION

The composition according to the present invention, when administered into body, isolates virus from the outside by using salt of polylactic acid and amphiphilic block copolymer, and thereby can increase the stability of virus in blood or body fluid. In addition, the composition according to the present invention can deliver virus into the targeted living tissue efficiently. Furthermore, the amphiphilic block copolymer has good biodegradability and biocompatibility.

### BRIEF EXPLANATION OF THE DRAWINGS

Figure 1 shows a diagram for a schematic structure of polymer nanoparticle delivery system prepared by a preparation method according to an embodiment of the present invention.
Figure 2 shows photographs measuring expression of Luciferase gene by luminescence measurement imaging system in order to confirm the gene absorption ratio in liver tissue of polymer nanoparticle delivery system according to an embodiment of the present invention.
Figure 3 shows photographs measuring luminescence generated by expression of Luciferase gene by luminescence measurement imaging system in Ex vivo form in order to confirm the gene absorption ratio in targeted living tissues of polymer nanoparticle delivery system according to an embodiment of the present invention.
Figure 4 shows graphs comparing the toxicity in liver tissue of polymer nanoparticle delivery system according to an embodiment of the present invention.
Figure 5 shows graphs comparing the anticancer efficacy in liver tissue of polymer nanoparticle delivery system according to an embodiment of the present invention.

### CONCRETE MODE FOR CARRYING OUT THE INVENTION

The present invention will be explained in detail below.

### Virus

The virus used in the composition for delivering virus of the present invention as defined in appended claim 1 is a virus for treating or preventing disease, and is an effective ingredient of the finally prepared composition.

In an embodiment, the virus for treating disease can be oncolytic virus. Example of the oncolytic virus is one or more selected from the group consisting of adenovirus, vaccinia virus, herpes simplex virus (HSV) and vesicular stomatitis virus (VSV). In an embodiment, the oncolytic virus is adenovirus. Adenovirus used in an embodiment of the present invention comprises Luciferase, and it can be confirmed through imaging.

The virus for treatment can express several kinds of treatment genes in the body of the subject. The virus for prevention can induce immunity to the target disease in the body of the subject. A composition comprising virus for preventing disease according to an example of the present invention can reduce immunity induction due to the virus itself, designate or extend the target cell, and reduce hyperimmune reaction to the virus when administrated again and thereby provide advantage of obtaining significant effect by inoculation for several times.

In an example of the present invention, the virus can be comprised preferably in an amount of 0.001 to 10 % by weight, and more concretely 0.01 to 5 % by weight, based on the total weight of the finally prepared composition. If the amount of virus is less than 0.001 % by weight, the amount of delivery system used becomes too much as compared with the drug, and thus there may be a side effect due to the delivery system. If the amount of virus is greater than 10 % by weight, the size of nanoparticle becomes too large, and thus the stability of nanoparticle may be lowered and the rate of loss during filter sterilization may increase.

### Amphiphilic block copolymer

The amphiphilic block copolymer used in the composition for delivering virus of the present invention is an A-B type block copolymer comprising a hydrophilic A block and a hydrophobic B block. The A-B type block copolymer is a core-shell type wherein in an aqueous phase the hydrophobic B block forms the core (inner wall) and the hydrophilic A block forms the shell (outer wall), and can control the in vivo distribution of the polymer delivery system or increase the efficiency of delivery of the system into cells.

The hydrophilic A block is one or more selected from the group consisting of monomethoxy polyethylene glycol, monoacetoxy polyethylene glycol, polyethylene glycol, a copolymer of polyethylene and propylene glycol, and polyvinyl pyrrolidone. In an embodiment, the hydrophilic A block may have a number average molecular weight of 200 to 50,000 Daltons, more concretely 1,000 to 20,000 Daltons, and still more concretely 1,000 to 5,000 Daltons.

In addition, if necessary, a functional group, a ligand, or a functional group capable of promoting intracellular delivery may be chemically bound to the end of the hydrophilic A block so as to control the in vivo distribution of the polymer nanoparticle delivery system formed by the amphiphilic block copolymer and the salt of polylactic acid, or to increase the efficiency of delivery of the nanoparticle delivery system into cells. The functional group or ligand may be one or more selected from the group consisting of monosaccharides, polysaccharides, vitamins, peptides, proteins, and antibodies to cell surface receptors. More concretely, the functional group or ligand may be one or more selected from the group consisting of anisamide, vitamin B9 (folic acid), vitamin B12, vitamin A, galactose, lactose, mannose, hyaluronic acid, RGD peptide, NGR peptide, transferrin, and antibody to transferrin receptor.

The hydrophobic B block is a biocompatible and biodegradable polymer, and is one or more selected from the group consisting of polyester, polyanhydride, polyamino acid, polyorthoester and polyphosphazine. More concretely, the hydrophobic B block may be one or more selected from the group consisting of polylactide, polyglycolide, polycaprolactone, polydioxane-2-one, a copolymer of polylactide and glycolide, a copolymer of polylactide and polydioxane-2-one, a copolymer of polylactide and polycaprolactone, and a copolymer of polyglycolide and polycaprolactone. In another embodiment, the hydrophobic B block may have a number average molecular weight of 50 to 50,000 Daltons, more concretely 200 to 20,000 Daltons, and still more concretely 1,000 to 5,000 Daltons. Also, in another embodiment, to improve the stability of the nanoparticle by increasing hydrophobicity of the hydrophobic B block, the hydroxyl group at the end of the hydrophobic B block may be modified with one or more selected from the group consisting of tocopherol, cholesterol, and C₁₀₋₂₄ fatty acid.

The amount of the amphiphilic block copolymer comprising the hydrophilic block (A) and the hydrophobic block (B) is 1 to 99.98 % by weight, and preferably, it may be concretely 10 to 99.8 % by weight, and more concretely 20 to 80 % by weight, based on the total dry weight of the composition. If the amount of the amphiphilic block copolymer is less than 1 % by weight, the size of the nanoparticle becomes too large, and thus the stability of the nanoparticle may be lowered and the rate of loss during filter sterilization may increase. If the amount of the amphiphilic block copolymer is greater than 99.98 % by weight, the amount of virus that can be incorporated may become too small.

Furthermore, regarding the compositional ratio of the hydrophilic block (A) and hydrophobic block (B) in the amphiphilic block copolymer, the amount of the hydrophilic block (A) may be 30 to 80 % by weight, more concretely 40 to 70 % by weight, based on the weight of the copolymer. If the amount of the hydrophilic block (A) is less than 30 % by weight, solubility of the polymer in water is low, and thus it may be difficult to form a nanoparticle. Thus, it is advantageous that the amount of the hydrophilic block (A) is 30 % by weight or greater so that the copolymer can have a solubility in water sufficient to form a nanoparticle. If the amount of the hydrophilic block (A) is greater than 80 % by weight, hydrophilicity becomes too high and thus the stability of the polymer nanoparticle may be lowered and it may be difficult to use as a composition for solubilizing the complex. Thus, considering stability of the nanoparticle, it is advantageous that the amount of the hydrophilic block (A) is 80 % by weight or less.

### Salt of polylactic acid

The salt of polylactic acid (e.g. PLANa) used in the composition for delivering virus of the present invention is distributed in the core (inner wall) of the nanoparticle, and acts to stabilize the nanoparticle by strengthening the hydrophobicity of the core, and at the same time, to effectively avoid reticuloendothelial system (RES) in the body. That is, the carboxylic anion in the salt of polylactic acid binds to the virus more efficiently than a polylactic acid, and decreases the surface potential of the polymer nanoparticle. Thereby, positive charge of the surface potential of the polymer nanoparticle becomes less than that of a polymer nanoparticle which does not contain a salt of polylactic acid, and thus it may be less captured by reticuloendothelial system and efficiently delivered to target sites (e.g., cancer cells, or inflammatory cells).

The salt of polylactic acid-which is contained as a separate ingredient from the amphiphilic block copolymer-is a component of the inner wall of the nanoparticle, and may have a number average molecular weight of 500 to 50,000 Daltons, and more concretely 1,000 to 10,000 Daltons. If the molecular weight of the salt of polylactic acid is less than 500 Daltons, the hydrophobicity becomes too low and thus the salt of polylactic acid may not easily exist at the core (inner wall) of the nanoparticle. If the molecular weight of the salt of polylactic acid is greater than 50,000 Daltons, the size of the polymer nanoparticle may become too large.

The salt of polylactic acid may be used in an amount of 1 to 500 parts by weight, more concretely 20 to 400 parts by weight, and still more concretely 40 to 300 parts by weight, based on 100 parts by weight of the amphiphilic block copolymer. If the amount of the salt of polylactic acid is greater than 500 parts by weight based on 100 parts by weight of the amphiphilic block copolymer, the size of the nanoparticle increases and thus the filtration using sterilization membrane may become difficult. If the amount of the salt of polylactic acid is less than 1 part by weight based on 100 parts by weight of the amphiphilic block copolymer, it is hard to obtain the desired effect.

In an embodiment, the composition of the present invention may comprise 1 to 2,000 parts by weight of the amphiphilic block copolymer and 1 to 1,000 parts by weight of the salt of polylactic acid, based on 1 part by weight of the virus. Preferably, the amphiphilic block copolymer may be contained in an amount of 5 to 1,000 parts by weight, and more preferably 10 to 500 parts by weight. Preferably, the salt of polylactic acid may be contained in an amount of 5 to 500 parts by weight, and more preferably 10 to 250 parts by weight.

In an embodiment, the end of the salt of polylactic acid opposite to the end of carboxylic acid-metal (e.g., sodium) may be substituted with one selected from the group consisting of hydroxyl, acetoxy, benzoyloxy, decanoyloxy, palmitoyloxy, and C₁₋₂ alkoxy.

The salt of polylactic acid in the present invention is one or more selected from the group consisting of the compounds of the following Formulas 1 to 6:

[Formula 1] **RO-CHZ-[A]ₙ-[B]ₘ-COOM**

In Formula 1 above, A is -COO-CHZ-; B is -COO-CHY-, -COO-CH₂CH₂CH₂CH₂CH₂- or -COO-CH₂CH₂OCH₂; R is a hydrogen atom, or acetyl, benzoyl, decanoyl, palmitoyl, methyl or ethyl; each of Z and Y is a hydrogen atom, or methyl or phenyl; M is Na, K or Li; n is an integer of from 1 to 30; and m is an integer of from 0 to 20.

[Formula 2] **RO-CHZ-[COO-CHX]ₚ-[COO-CHY']_{q}-COO-CHZ-COOM**

In Formula 2 above, X is methyl; Y' is a hydrogen atom or phenyl; p is an integer of from 0 to 25, q is an integer of from 0 to 25, with the proviso that p + q is an integer of from 5 to 25; R is a hydrogen atom, or acetyl, benzoyl, decanoyl, palmitoyl, methyl or ethyl; M is Na, K or Li; and Z is a hydrogen atom, methyl or phenyl.

[Formula 3] **RO-PAD-COO-W-M'**

In Formula 3 above, W-M' is 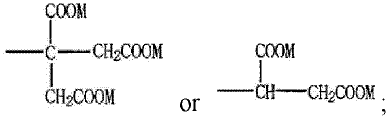 PAD is selected from the group consisting of D,L-polylactic acid, D-polylactic acid, polymandelic acid, copolymer of D,L-lactic acid and glycolic acid, copolymer of D,L-lactic acid and mandelic acid, copolymer of D,L-lactic acid and caprolactone, and copolymer of D,L-lactic acid and 1,4-dioxane-2-one; R is a hydrogen atom, or acetyl, benzoyl, decanoyl, palmitoyl, methyl or ethyl; and M is independently Na, K or Li.

[Formula 4] S-O-PAD-COO-Q

In Formula 4 above, S is 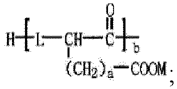 L is -NR₁- or -O-, wherein R₁ is a hydrogen atom or C₁₋₁₀ alkyl; Q is -CH₃, -CH₂CH₃, -CH₂CH₂CH₃, -CH₂CH₂CH₂CH₃, or -CH₂C₆H₅; a is an integer of from 0 to 4; b is an integer of from 1 to 10; M is Na, K or Li; and PAD is one or more selected from the group consisting of D,L-polylactic acid, D-polylactic acid, polymandelic acid, copolymer of D,L-lactic acid and glycolic acid, copolymer of D,L-lactic acid and mandelic acid, copolymer of D,L-lactic acid and caprolactone, and copolymer of D,L-lactic acid and 1,4-dioxane-2-one.

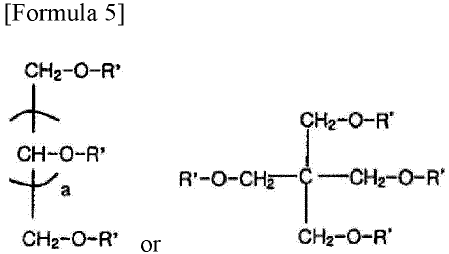

In Formula 5 above, R' is -PAD-O-C(O)-CH₂CH₂-C(O)-OM, wherein PAD is selected from the group consisting of D,L-polylactic acid, D-polylactic acid, polymandelic acid, copolymer of D,L-lactic acid and glycolic acid, copolymer of D,L-lactic acid and mandelic acid, copolymer of D,L-lactic acid and caprolactone, and copolymer of D,L-lactic acid and 1,4-dioxane-2-one, M is Na, K or Li; and a is an integer of from 1 to 4.

[Formula 6] **YO-[-C(O)-(CHX)ₐ-O-]ₘ-C(O)-R-C(O)-[-O-(CHX')_{b}-C(O)-]ₙ-OZ**

In Formula 6 above, X and X' are independently hydrogen, C₁₋₁₀ alkyl or C₆₋₂₀ aryl; Y and Z are independently Na, K or Li; m and n are independently an integer of from 0 to 95, with the proviso that 5 < m + n < 100; a and b are independently an integer of from 1 to 6; and R is - (CH₂)ₖ-, C₂₋₁₀ divalent alkenyl, C₆₋₂₀ divalent aryl or a combination thereof, wherein k is an integer of from 0 to 10.

The salt of polylactic acid is preferably the compound of Formula 1 or Formula 2.

### Cationic compound

In an embodiment, the composition for delivering virus of the present invention may further comprise cationic compound.

That is, in the present invention the amphiphilic block copolymer and the salt of polylactic acid form nanoparticle structure, inside of which the virus is entrapped, and according to an embodiment, this nanoparticle structure may further comprise cationic compound.

The cationic compound is combined with the negatively charged outer shell of the virus by electrostatic interaction, and thereby it can contribute to stabilization of the virus within the nanoparticle structure. The virus can be combined simultaneously with the hydrophobic parts of the amphiphilic block copolymer and the salt of polylactic acid.

The cationic compound includes any type of compound capable of forming a complex with the virus by electrostatic interaction, and for example, it may be cationic lipids and polymers.

The cationic lipid may be one or a combination of two or more selected from the group consisting of N,N-dioleyl-N,N-dimethylammoniumchloride (DODAC), N,N-distearyl-N,N-dimethylammoniumbromide (DDAB), N-(1-(2,3-dioleoyloxy)propyl-N,N,N-trimethylammoniumchloride (DOTAP), N,N-dimethyl-(2,3-dioleoyloxy)propylamine (DODMA), N,N,N-trimethyl-(2,3-dioleoyloxy)propylamine (DOTMA), 1,2-diacyl-3-trimethylammonium-propane (TAP), 1,2-diacyl-3-dimethylammonium-propane (DAP), 3β-[N-(N',N',N'-trimethylaminoethane)carbamoyl]cholesterol (TC-cholesterol), 3β-[N-(N',N'-dimethylaminoethane)carbamoyl] cholesterol (DC-cholesterol), 3β-[N-(N'-monomethylaminoethane)carbamoyl] cholesterol (MC-cholesterol), 3β-[N-(aminoethane)carbamoyl]cholesterol (AC-cholesterol), cholesteryloxypropane-1-amine (COPA), N-(N'-aminoethane)carbamoylpropanoic tocopherol (AC-tocopherol) and N-(N'-methylaminoethane)carbamoylpropanoic tocopherol (MC-tocopherol). If such a cationic lipid is used, it is preferable to use polycationic lipid having high cation density as less as possible in order to decrease toxicity induced by the cationic lipid, and more concretely, the number of the functional group in a molecule which is capable of exhibiting positive charge in an aqueous solution may be one.

In a more preferable embodiment, the cationic lipid may be one or more selected from the group consisting of 3β-[N-(N',N',N'-trimethylaminoethane)carbamoyl]cholesterol (TC-cholesterol), 3β-[N-(N',N'-dimethylaminoethane)carbamoyl]cholesterol (DC-cholesterol), 3β-[N-(N'-monomethylaminoethane)carbamoyl]cholesterol (MC-cholesterol), 3β-[N-(aminoethane)carbamoyl]cholesterol (AC-cholesterol), N-(1-(2,3-dioleoyloxy)propyl-N,N,N-trimethylammoniumchloride (DOTAP), N,N-dimethyl-(2,3-dioleoyloxy)propylamine (DODMA) and N,N,N-trimethyl-(2,3-dioleoyloxy)propylamine (DOTMA).

In addition, the cationic lipid may be a lipid having several functional groups which are capable of exhibiting positive charge in an aqueous solution. Concretely, it may be one or more selected from the group consisting of N,N-dioleyl-N,N-dimethylammoniumchloride (DODAC), N,N-distearyl-N,N-dimethylammoniumbromide (DDAB), 1,2-diacyl-3-trimethylammonium-propane (TAP), 1,2-diacyl-3-dimethylammonium-propane (DAP).

In a concrete embodiment, the cationic lipid may be represented by the following Formula A:

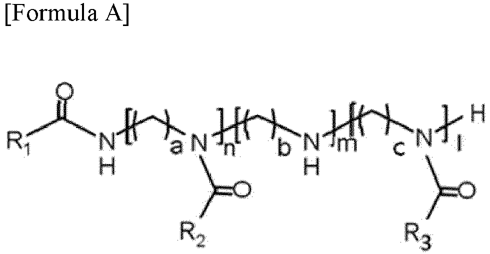

In the above formula,
each of n, m and 1 is 0 to 12 with the proviso that 1 ≤ n + m + 1 ≤ 12; each of a, b and c is 1 to 6; and each of R₁, R₂ and R₃ is independently hydrogen or a saturated or unsaturated C₁₁₋₂₅ hydrocarbon with the proviso that at least one of R₁, R₂ and R₃ is a saturated or unsaturated C₁₁₋₂₅ hydrocarbon.

Preferably, n, m and l may be independently 0 to 7, and 1 ≤ n + m + 1 ≤ 7.

Preferably, a, b and c may be 2 to 4.

Preferably, each of R₁, R₂ and R₃ may be independently selected from the group consisting of lauryl, myristyl, palmityl, stearyl, arachidyl, behenyl, lignoceryl, cerotyl, myristoleyl, palmitoleyl, sapienyl, oleyl, linoleyl, arachidonyl, eicosapentaenyl, erucyl, docosahexaenyl and cerotyl.

Concrete example of the cationic lipid may be one or more selected from the group consisting of 1,6-dioleoyl triethylenetetramide(N,N'-((ethane-1,2-diylbis(azanediyl))bis(ethane-2,1-diyl))dioleamide), 1,8-dilinoleoyl tetraethylenepentamide ((9Z,9'Z,12Z,12'Z)-N,N'-(((azanediylbis(ethane-2,1-diyl))bis(azanediyl))bis(ethane-2,1-diyl))bis(octadeca-9,12-dienamide)), 1,4-dimyristoleoyl diethylenetriamide ((9Z,9'Z)-N,N'-(azanediylbis(ethane-2,1-diyl))bis(tetradec-9-enamide)), 1,10-di stearoyl pentaethylenehexamide (N,N'-(3,6,9,12-tetraazatetradecane-1,14-diyl)distearamide), and 1,10-dioleoyl pentaethylenehexamide (N,N'-(3,6,9,12-tetraazatetradecane-1,14-diyl)dioleamide).

On the other hand, the cationic polymer may be selected from the group consisting of chitosan, glycol chitosan, protamine, polylysine, polyarginine, polyamidoamine (PAMAM), polyethylenimine, dextran, hyaluronic acid, albumin, polyethylenimine (PEI), polyamine and polyvinylamine (PVAm), and preferably it may be one or more selected from polyethylenimine (PEI), polyamine and polyvinylamine (PVA).

The cationic compound used in the present invention may be used in an amount of 0.01 to 50 % by weight, more concretely 0.1 to 20 % by weight, based on the total weight of the finally prepared composition. If the amount of the cationic compound is less than 0.01 % by weight, it may not be sufficient to entrap the virus. If the amount of the cationic compound is greater than 50 % by weight, the size of the nanoparticle becomes too large, and thus the stability of the nanoparticle may be lowered and the rate of loss during filter sterilization may increase.

In a concrete embodiment, the amount of the cationic compound used, based on 1 X 10¹⁰ VP of virus, may be preferably 0.1 to 40 µg, concretely 0.5 to 35 µg, more concretely 1 to 30 µg, still more concretely 1 to 25 µg, and most concretely 6 to 24 µg. If the amount of the cationic compound is less than 0.1 µg, the cationic compound may not sufficiently entrap the virus. Thus, it is advantageous that the amount of the cationic compound is 0.1 µg or greater so that a complex containing a sufficient amount of virus can be formed by the electrostatic binding of the cationic compound and the virus. In contrast, the amount of the cationic compound may be preferably 40 µg or less since if the amount is greater than 40 µg, toxicity may be caused.

### Divalent or trivalent metal ion

In an embodiment, the composition for delivering virus of the present invention may further comprise divalent or trivalent metal ion.

The divalent or trivalent metal ion may be preferably selected from calcium (Ca²⁺), magnesium (Mg²⁺), barium (Ba²⁺), chromium (Cr³⁺), iron (Fe³⁺), manganese (Mn²⁺), nickel (Ni²⁺), copper (Cu²⁺), and zinc (Zn²⁺) or aluminum (Al³⁺).

The divalent or trivalent metal ion may be added to the polymer nanoparticle composition in a form of sulfate salt, chloride salt, carbonate salt, phosphate salt or hydroxide. Preferably, it may be added in a form of calcium chloride (CaCl₂), magnesium chloride (MgCl₂), zinc chloride (ZnCl₂), aluminum chloride (AlCl₃), ferric chloride (FeCl₃), calcium carbonate (CaCO₃), magnesium carbonate (MgCO₃), calcium phosphate (Ca₃(PO₄)₂), magnesium phosphate (Mg₃(PO₄)₂), aluminum phosphate (AlPO₄), magnesium sulfate (MgSO₄), calcium hydroxide (Ca(OH)₂), magnesium hydroxide (Mg(OH)₂), aluminum hydroxide (Al(OH)₃), zinc hydroxide (Zn(OH)₂) or a mixture thereof.

By controlling the amount of equivalent of the divalent or trivalent metal ion, the release rate of drug entrapped in the polymer nanoparticle can be controlled. Concretely, if the divalent or trivalent metal ion is contained in the polymer nanoparticle composition in an amount of 1 equivalent or less to the equivalent of the carboxyl group of the salt of polylactic acid, the number thereof binding to the carboxyl terminal group of the salt of polylactic acid is small and the release rate of drug increases, and if it is contained in an amount of 1 equivalent or more, the number thereof binding to the carboxyl terminal group of the salt of polylactic acid is large and the drug release is sustained. Thus, in order to increase the release rate of drug in blood, less equivalent of metal ion may be used, whereas in order to sustain the drug release, more equivalent of metal ion may be used.

Also, the divalent or trivalent metal ion may be contained in amount of 0.01 to 10 equivalents, 0.1 to 5 equivalents or 0.2 to 2 equivalents, to the equivalent of the carboxyl terminal group of the salt of polylactic acid.

### Method for preparing a composition for delivering virus

Another aspect of the present invention provides a method for preparing a composition for delivering virus as defined in appended claim 10, comprising: (a) a step of dissolving virus in aqueous solvent; (b) a step of dissolving each of amphiphilic block copolymer and salt of polylactic acid in organic solvent; and (c) a step of mixing the solution of the steps (a) and (b) to form an emulsion.

The above steps (a) to (c) are steps for preparing a complex of amphiphilic block copolymer and salt of polylactic acid by dissolving virus in aqueous solvent and each of amphiphilic block copolymer and salt of polylactic acid in organic solvent, and mixing them to prepare an emulsion in monophase system.

In the above step (a), the aqueous solvent used may be distilled water, water for injection, or buffer, and a preferable buffer may be phosphate buffered saline.

In the above step (b), the organic solvent used may be water-miscible organic solvent, and may be, for example, C1 to C5 lower alcohol (including methanol, ethanol, and propanol), acetone, ethyl acetate or mixture thereof.

In the above step (b), each of the amphiphilic block copolymer and the salt of polylactic acid is dissolved in organic solvent, and the organic solvent used at this time may be one or more selected from the group consisting of acetone, ethanol, methanol, methylene chloride, chloroform, dioxane, dimethyl sulfoxide, acetonitrile, ethyl acetate and acetic acid. Preferably, it may be one or more selected from the group consisting of ethanol, ethyl acetate and acetic acid. The amount of organic solvent used can be properly adjusted and it can be used for dissolution of the amphiphilic block copolymer and the salt of polylactic acid.

In an embodiment of the present invention, the above step (b) may further comprise a step of dissolving cationic compound in organic solvent.

In the above step (c), an aqueous solution of virus obtained in step (a), an organic solution of amphiphilic block copolymer and an organic solution of salt of polylactic acid, and optionally an organic solution of cationic compound obtained in step (b) are mixed to form an emulsion. For example, based on volume, the ratio of the organic solution of amphiphilic block copolymer, salt of polylactic acid and optionally cationic compound to the aqueous solution of virus (the organic solution of amphiphilic block copolymer, salt of polylactic acid and optionally cationic compound / the aqueous solution of virus) may be 1 to 30, and more concretely 2 to 20. The solutions are mixed through suitable mixing means known in this field of art, and an example of such means may be ultrasonicator.

As an additional embodiment, the method for preparing a composition for delivering virus according to the present invention may further comprise: (d) a step of selectively removing the organic solvent from the mixture obtained in step (c).

Preferably, in the above step (d), the organic solvent is removed from the mixture containing stabilized nanoparticle prepared in step (c) by various removal methods, for example, organic solvent evaporation, to obtain an aqueous solution of polymer nanoparticle. In addition, the organic solvent may be diluted and removed by dialysis using osmotic membrane.

As a preferable embodiment, the method of the present invention may further comprise: (e) a step of adding divalent or trivalent metal ion, after the above step (d).

Furthermore, as a preferable embodiment, the method of the present invention may further comprise: (f) a step of lyophilization with addition of lyophilization aid, after the above step (e).

As a further additional embodiment, the method of the present invention may further comprise: a step of sterilizing the aqueous solution of polymer nanoparticle obtained in the above step (e) with sterilization filter, before the lyophilization of the above step (f).

The lyophilization aid used in the present invention is added to allow the lyophilized composition to maintain a cake form, or to help uniform dissolution of the composition of amphiphilic block copolymer, and salt of polylactic acid, as defined in the claims in short time during the course of reconstitution after lyophilization. Concretely, the lyophilization aid may be one or more selected from the group consisting of lactose, mannitol, sorbitol and sucrose. The amount of the lyophilization aid may be 1 to 90 % by weight, and more concretely 4 to 20 % by weight, based on the total dry weight of the lyophilized composition.

By a method according to an embodiment of the present invention, the virus, amphiphilic block copolymer and salt of polylactic acid are emulsified in an aqueous phase which is monophase system. The monophase system means a system which does not include phase separation by using one solvent or mixable solvents in the production process. If a monophase system is used, a complex in nanoparticle form is effectively formed by hydrophobic binding, and the binding force increases in the procedure of removing aqueous solution through lyophilization, and the yield of the polymer nanoparticle prepared finally becomes improved significantly. In addition, since such a method uses relatively less organic solvent, it is eco-friendly, reproducible, and easy for production, and advantageous for mass production by changing to hydrophobic drug particle though virus complex formation. Furthermore, since the organic solvent is used in relatively less amount, an effect of reducing toxicity due to organic solvent in case of in vivo application can be expected.

Also, in a composition prepared according to an embodiment of the present invention, the virus maintains the state of being entrapped in nanoparticle structure formed by the amphiphilic block copolymer and salt of polylactic acid, and thus the safety and stability in blood or body fluid are improved.

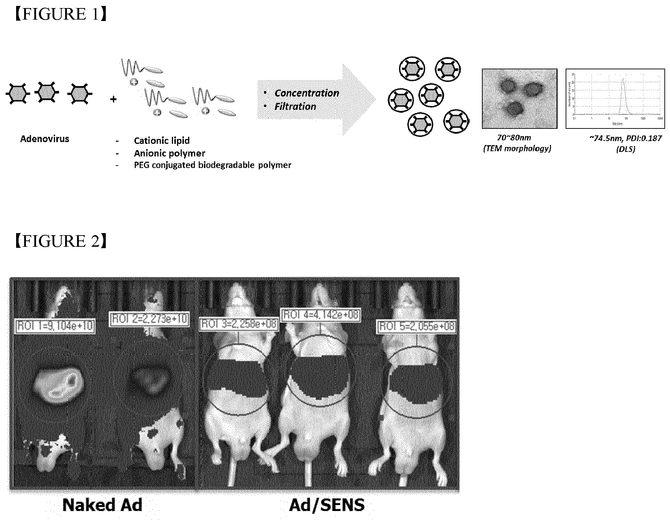

According to a preparation method of an embodiment of the present invention, the virus and the salt of polylactic acid bind to each other through hydrophobic interaction to form a complex, and the complex is entrapped in the nanoparticle structure formed by the amphiphilic block copolymer, resulting in polymer nanoparticle structure. A schematic structure of polymer nanoparticle delivery system prepared by a preparation method according to an embodiment of the present invention as such is shown in Figure 1. The matters relating to the virus, amphiphilic block copolymer, etc. as constitutional components of the composition are the same as described above.

An embodiment of the present invention may further comprise divalent or trivalent metal ion in order for the polymer nanoparticle to have more improved stability in aqueous solution. The divalent or trivalent metal ion binds to the carboxyl terminal group of the salt of polylactic acid in the polymer nanoparticle. The divalent or trivalent metal ion forms metal ion bonding by substitution reaction with the monovalent metal cation of the carboxyl terminal group of the salt of polylactic acid in the polymer nanoparticle. The formed metal ion bonding has a stronger bonding force, and forms more stable polymer nanoparticle.

In a preferable embodiment, the particle size of the nanoparticle in the composition is preferably 10 to 300 nm, and more concretely 10 to 150 nm. In addition, the standard charge of the nanoparticle is preferably -40 to 10 mV, and more concretely -30 or 0 mV. The particle size and the standard charge are most preferable in terms of the stability of the nanoparticle structure, and the amounts of the constitutional components and in vivo absorption and stability of the virus.

The composition containing virus-salt of polylactic acid entrapped in nanoparticle structure of amphiphilic block copolymer according to the present invention may be administered in the route of blood vessel, muscle, subcutaneous, oral, bone, transdermal or local tissue and it may be formulated into various formulations for oral or parenteral administration to be suitable for such administration routes. Examples of the formulation for oral administration may include tablet, capsule, powder, or liquid, and the examples of the formulation for parenteral administration may include eye drop, or injection. As a preferred embodiment, the composition may be a formulation for injection, and more preferably a formulation for intravenous injection. For example, in case of lyophilizing the composition according to the present invention, it may be prepared in a form of formulation for injection by reconstituting it with distilled water for injection, 0.9% physiological saline, or 5% dextrose aqueous solution.

The present invention will be explained below in more detail with reference to the following Examples.

### EXAMPLES

### [Comparative Example 1] Adenovirus vector

1 X 10¹⁰ VP of wild-type adenovirus expressing luciferase gene of SEQ ID NO: 1 was dissolved in 10 µl of PBS to prepare a composition (referred to as `Naked Ad' hereinafter). The composition obtained in Comparative Example 1 was that as shown in the following Table 1.

**[Table 1]**

| | Composition | Ad | mPEG-PLA | PLA-Na |
|---|---|---|---|---|
| Comparative Example 1 | Naked Ad | 1 X 10¹⁰ VP | - | - |

### [Comparative Example 2] Preparation of composition containing adenovirus plasmid DNA (Ad pDNA)/1,6-dioleoyl triethylenetetramide (dio-TETA)/mPEG-PLA tocopherol (2k-1.7k)/dioleoyl phosphatidyl-ethanolamine (DOPE)

A solution of 1 µg of plasmid DNA having 35,000 base pairs expressing luciferase gene of SEQ ID NO: 1 dissolved in 4.35 µl of distilled water, a solution of 10.4 µg of dio-TETA dissolved in 10.4 µl of ethanol, a solution of 10.4 µg of DOPE dissolved in 10.4 µl of ethanol, a solution of 20 µg of mPEG-PLA-tocopherol (2k-1.7k) dissolved in 0.2 µl of ethanol, and a solution of 20 µg of PLA-Na dissolved in 2 µl of ethanol were mixed in this order and further mixed for 10 minutes in ultrasonicator (bath type). The prepared complex emulsion solution was put into 1-neck round bottom flask and distilled under reduced pressure in a rotary evaporator to selectively remove ethanol, and thereby to prepare a composition containing Ad pDNA/dioTETA/DOPE/mPEG-PLA-tocopherol (2k-1.7k)/PLA-Na (1.7k) (referred to as `Ad DNA/SENS' hereinafter). The prepared composition was filtered through 0.45 µm hydrophilic filter and then stored at 4°C, and in experimental procedures thereafter, it was mixed with 10X PBS to make 1X to the final volume. The composition obtained in Comparative Example 2 was that as shown in the following Table 2.

**[Table 2]**

| | Composition | Ad DNA | dio-TETA | DOPE | mPEG-PLA-tocopherol | PLA-Na |
|---|---|---|---|---|---|---|
| Comparative Example 2 | Ad pDNA/SENS | 1 µg | 10.4 µg | 10.4 µg | 20 µg | 20 µg |

### [Example 1] Preparation of composition containing adenovirus/mPEG-PLA (2k-1.7k)/PLA-Na (1.7k)

1 X 10¹⁰ VP of wild-type adenovirus expressing luciferase gene of SEQ ID NO: 1 was dissolved in 10 µl of PBS, and thereto a solution of 40 µg of mPEG-PLA (2k-1.7k) dissolved in 0.4 µl of ethanol and a solution of 100 µg of PLA-Na (1.7k) dissolved in 10 µl of ethanol were mixed in this order and further mixed for 10 minutes in ultrasonicator (bath type). The prepared complex emulsion solution was put into 1-neck round bottom flask and distilled under reduced pressure in a rotary evaporator to selectively remove ethanol, and thereby to prepare a composition containing Ad/mPEG-PLA(2k-1.7k)/PLA-Na (1.7k) (referred to as 'Ad-vSENS' hereinafter). The prepared composition was filtered through 0.45 µm hydrophilic filter and then stored at 4°C, and in experimental procedures thereafter, it was mixed with 10X PBS to make 1X to the final volume. The composition obtained in Example 1 was that as shown in the following Table 3.

**[Table 3]**

| | Composition | Ad | mPEG-PLA | PLA-Na |
|---|---|---|---|---|
| Example 1 | Ad-vSENS | 1 X 10¹⁰ VP | 40 µg | 100 µg |

### [Example 2] Preparation of composition containing adenovirus/mPEG-PLA (2k-1.7k)/PLA-Na (1.7k)/CaCl₂

1 X 10¹⁰ VP of wild-type adenovirus expressing luciferase gene of SEQ ID NO: 1 was dissolved in 10 µl of PBS, and thereto a solution of 40 µg of mPEG-PLA (2k-1.7k) dissolved in 0.4 µl of ethanol and a solution of 100 µg of PLA-Na (1.7k) dissolved in 10 µl of ethanol were mixed in this order and further mixed for 10 minutes in ultrasonicator (bath type). The prepared complex emulsion solution was put into 1-neck round bottom flask and distilled under reduced pressure in a rotary evaporator to selectively remove ethanol, and thereby to prepare a composition containing Ad/mPEG-PLA(2k-1.7k)/PLA-Na (1.7k). Then, a solution of 3.3 µg of CaCl₂ dissolved in 1.7 µl of PBS was added thereto (referred to as 'Ad-vSENS+CaCl₂' hereinafter). The prepared composition was filtered through 0.45 µm hydrophilic filter and then stored at 4°C, and in experimental procedures thereafter, it was mixed with 10X PBS to make 1X to the final volume. The composition obtained in Example 2 was that as shown in the following Table 4.

**[Table 4]**

| | Composition | Ad | mPEG-PLA | PLA-Na | CaCl₂ |
|---|---|---|---|---|---|
| Example 2 | Ad-vSENS+CaCl₂ | 1 X 10¹⁰ VP | 40 µg | 100 µg | 3.3 µg |

### [Example 3] Preparation of composition containing adenovirus/1,6-dioleoyl triethylenetetramide (dio-TETA)/PLA-Na (1.7k)/mPEG-PLA-tocopherol (2k-1.7k)

1 X 10¹⁰ VP of wild-type adenovirus expressing luciferase gene of SEQ ID NO: 1 was dissolved in 100 µl of PBS. A solution of 20 µg of dio-TETA dissolved in 2 µl of ethanol, a solution of 100 µg of PLA-Na (1.7k) dissolved in 2 µl of ethanol and a solution of 100 µg of mPEG-PLA-tocopherol (2k-1.7k) dissolved in 2 µl of ethanol were mixed in this order, and finally mixed with the previously prepared 100 µl PBS containing the virus to prepare a composition containing Ad/dio-TETA/PLA-Na (1.7k)/mPEG-PLA-tocopherol (2k-1.7k) (referred to as 'Ad-vSENS_2' hereinafter). The composition obtained in Example 3 was that as shown in the following Table 5.

**[Table 5]**

| | Composition | Ad | dio-TETA | PLA-Na (1.7k) | mPEG-PLA-tocopherol (2k-1.7k) |
|---|---|---|---|---|---|
| Example 3 | Ad-vSENS_2 | 1 X 10¹⁰ VP | 20 µg | 100 µg | 50 µg |

### [Experimental Example 1] Comparison of size and surface charge of composition according to formulation

In order to confirm whether or not nanoparticles were formed according to formulation, the size and surface charge were measured. The measurements of size and surface charge of particles were made by using dynamic light scattering (DLS) method. Concretely, He-Ne laser was used as a light source, and Zetasizer Nano ZS90 device (MALVERN) was operated according to the manual. The sizes and surface charges of the nanoparticles of Comparative Examples 1 and 2 and Examples 1 to 3 according to formulation are shown in the following Table 6.

**[Table 6]**

| | Kind of composition | Particle size (based on intensity) | Surface charge |
|---|---|---|---|
| Comparative Example 1 | Naked Ad | 119.8 nm | -16.8 mV |
| Comparative Example 2 | Ad pDNA/SENS | 152.3 nm | -10.7 mV |
| Example 1 | Ad-vSENS | 129.7 nm | -29.5 mV |
| Example 2 | Ad-vSENS+CaCl₂ | 125.3 nm | -28.2 mV |
| Example 3 | Ad-vSENS_2 | 191.8 nm | -2.81 mV |

### [Experimental Example 2] Comparison of uptake ratio in liver tissue according to formulation

In vivo gene expression levels were measured by using luciferase expression gene. In vivo bioluminescence values were measured by using IVIS spectrum in vivo imaging system method. IVIS LUMINA III device (PerkinElmer) was operated according to the manual. The liver uptake values and ratios of Comparative Example 1 and Examples 1 to 3 according to formulation are shown in the following Table 7 and Figure 2.

**[Table 7]**

| | Kind of composition | Liver | Liver uptake ratio (to Naked) |
|---|---|---|---|
| Comparative Example 1 | Naked Ad | 5.7 X 10¹⁰ | 1 |
| Example 1 | Ad-vSENS | 2.9 X 10⁸ | 0.005 |
| Example 2 | Ad-vSENS(+CaCl₂) | 2.6 X 10⁷ | 0.0005 |
| Example 3 | Ad-vSENS_2 | 4.3 X 10⁶ | 0.000075 |

### [Experimental Example 3] Comparison of expression efficiency in tissue according to formulation

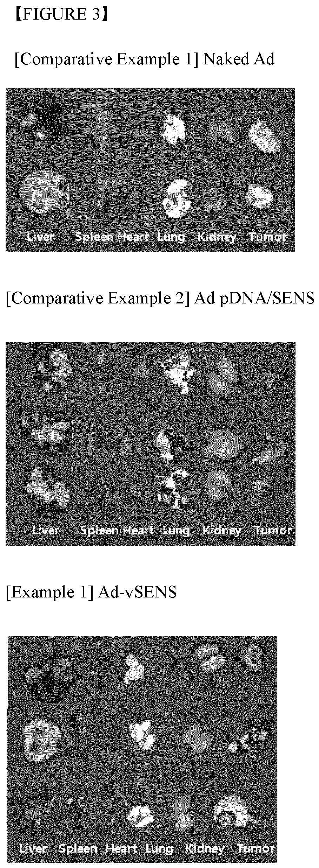

Ex vivo gene expression levels were measured by using luciferase expression gene. Ex vivo bioluminescence values were measured by using IVIS spectrum in vivo imaging system method. IVIS LUMINA III device (PerkinElmer) was operated according to the manual. The distributions of gene expression for each organ of Comparative Examples 1 and 2 and Example 1 according to formulation are shown in the Figure 3.

### [Experimental Example 4] Comparison of toxicity in liver tissue according to formulation

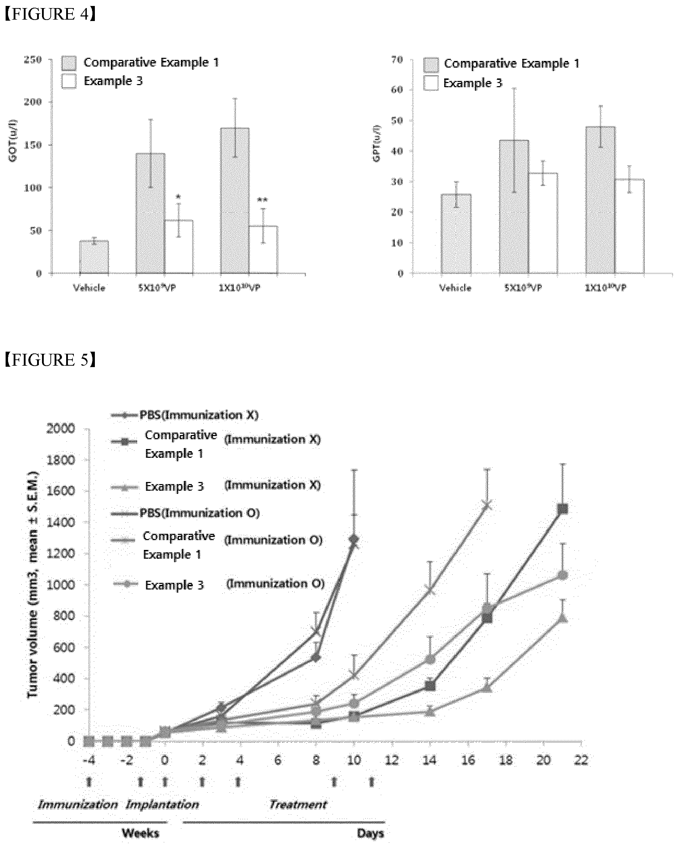

All tested materials were administered one time through intravenous administration (i.v.). In case of comparison tests for toxicity in liver tissue, the serum was extracted and analyzed at 72 hours after the administration. The compared values of toxicity in liver tissue of Comparative Example 1 and Example 3 according to formulation are shown in the Figure 4.

### [Experimental Example 5] Comparison of anticancer efficacy after immunization according to formulation

All tested materials were administered one time through intravenous administration (i.v.). For comparison according to immunization, the experiments were divided and conducted for the immunized test group and the non-immunized control group. For immunization, total of 2 administrations of virus were made for 4 weeks with 2 weeks' interval. In case of Comparative Example 1 and Example 3, total of 5 administrations were made for 11 days with 2 days' interval. In case of negative control material, the same dose was administered in the same manner. In case of intravenous administration, the animal was carefully put into a retaining appliance, and then intravenous administration was made to caudal vein by using a syringe equipped with 26 gauge needle. The values of tumor size change exhibiting anticancer efficacy of Comparative Example 1 and Example 3 according to formulation are shown in the Figure 5.

## Claims

1. A composition for delivering virus, comprising: virus as effective ingredient; amphiphilic block copolymer; and salt of polylactic acid;
wherein the virus is entrapped in a nanoparticle structure formed by the amphiphilic block copolymer and the salt of polylactic acid
wherein the amphiphilic block copolymer is an A-B type block copolymer comprising a hydrophilic A block and a hydrophobic B block, wherein the hydrophilic A block is one or more selected from the group consisting of monomethoxy polyethylene glycol, monoacetoxy polyethylene glycol, polyethylene glycol, a copolymer of polyethylene and propylene glycol, and polyvinyl pyrrolidone, and the hydrophobic B block is one or more selected from the group consisting of polyester, polyanhydride, polyamino acid, polyorthoester and polyphosphazine; and
wherein the salt of polylactic acid is one or more selected from the group consisting of the compounds of the following Formulas 1 to 6:
[Formula 1] **RO-CHZ-[A]ₙ-[B]ₘ-COOM**
wherein A is -COO-CHZ-; B is -COO-CHY-, -COO-CH₂CH₂CH₂CH₂CH₂- or -COO-CH₂CH₂OCH₂-; R is a hydrogen atom, or acetyl, benzoyl, decanoyl, palmitoyl, methyl or
ethyl; each of Z and Y is a hydrogen atom, or methyl or phenyl; M is Na, K or Li; n is an integer of from 1 to 30; and m is an integer of from 0 to 20;
[Formula 2] **RO-CHZ-[COO-CHX]ₚ-[COO-CHY']_{q}-COO-CHZ-COOM**
wherein X is methyl; Y' is a hydrogen atom or phenyl; p is an integer of from 0 to 25, q is an integer of from 0 to 25, with the proviso that p + q is an integer of from 5 to 25; R is a hydrogen atom, or acetyl, benzoyl, decanoyl, palmitoyl, methyl or ethyl; M is Na, K or Li; and Z is a hydrogen atom, methyl or phenyl;
[Formula 3] **RO-PAD-COO-W-M'**
wherein W-M' is 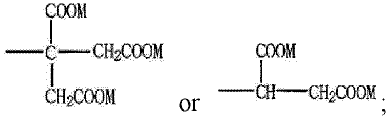 PAD is selected from the group consisting of D,L-polylactic acid, D-polylactic acid, polymandelic acid, copolymer of D,L-lactic acid and glycolic acid, copolymer of D,L-lactic acid and mandelic acid, copolymer of D,L-lactic acid and caprolactone, and copolymer of D,L-lactic acid and 1,4-dioxane-2-one; R is a hydrogen atom, or acetyl, benzoyl, decanoyl, palmitoyl, methyl or ethyl; and M is independently Na, K or Li;
[Formula 4] **S-O-PAD-COO-Q**
wherein S is 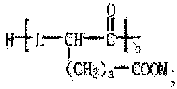 L is -NR₁- or -O-, wherein R₁ is a hydrogen atom or C₁₋₁₀ alkyl; Q is -CH₃, -CH₂CH₃, -CH₂CH₂CH₃, -CH₂CH₂CH₂CH₃, or -CH₂C₆H₅; a is an integer of from 0 to 4; b is an integer of from 1 to 10; M is Na, K or Li; and PAD is one or more selected from the group consisting of D,L-polylactic acid, D-polylactic acid, polymandelic acid, copolymer of D,L-lactic acid and glycolic acid, copolymer of D,L-lactic acid and mandelic acid, copolymer of D,L-lactic acid and caprolactone, and copolymer of D,L-lactic acid and 1,4-dioxane-2-one; 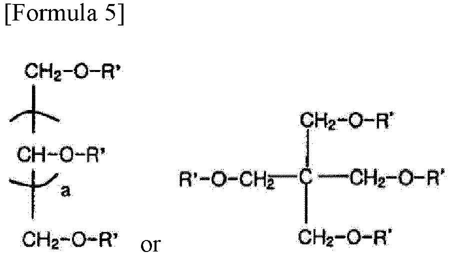
wherein R' is -PAD-O-C(O)-CH₂CH₂-C(O)-OM, wherein PAD is selected from the group consisting of D,L-polylactic acid, D-polylactic acid, polymandelic acid, copolymer of D,L-lactic acid and glycolic acid, copolymer of D,L-lactic acid and mandelic acid,
copolymer of D,L-lactic acid and caprolactone, and copolymer of D,L-lactic acid and 1,4-dioxane-2-one, M is Na, K or Li; and a is an integer of from 1 to 4;
[Formula 6] **YO-[-C(O)-(CHX)ₐ-O-]ₘ-C(O)-R-C(O)-[-O-(CHX')_{b}-C(O)-]ₙ-OZ**
wherein X and X' are independently hydrogen, C₁₋₁₀ alkyl or C₆₋₂₀ aryl; Y and Z are independently Na, K or Li; m and n are independently an integer of from 0 to 95, with the proviso that 5 < m + n < 100; a and b are independently an integer of from 1 to 6; and R is -(CH₂)ₖ-, C₂₋₁₀ divalent alkenyl, C₆₋₂₀ divalent aryl or a combination thereof, wherein k is an integer of from 0 to 10.

2. The composition for delivering virus of Claim 1, wherein the virus is oncolytic virus.

3. The composition for delivering virus of Claim 2, wherein the oncolytic virus is one or more selected from the group consisting of adenovirus, vaccinia virus, herpes simplex virus (HSV) and vesicular stomatitis virus (VSV).

4. The composition for delivering virus of Claim 1, wherein a hydroxyl group at the end of the hydrophobic B block is modified by one or more selected from the group consisting of tocopherol, cholesterol, and C₁₀₋₂₄ fatty acid.

5. The composition for delivering virus of Claim 1, wherein the salt of polylactic acid is a compound of the Formula 1 or 2.

6. The composition for delivering virus of Claim 1, further comprising cationic compound.

7. The composition for delivering virus of Claim 1, further comprising divalent or trivalent metal ion.

8. The composition for delivering virus of Claim 7, wherein the divalent or trivalent metal ion is one or more selected from the group consisting of calcium (Ca²⁺), magnesium (Mg²⁺), barium (Ba²⁺), chromium (Cr³⁺), iron (Fe³⁺), manganese (Mn²⁺), nickel (Ni²⁺), copper (Cu²⁺), zinc (Zn²⁺) and aluminum (Al³⁺).

9. The composition for delivering virus of Claim 7, wherein the divalent or trivalent metal ion is comprised in form of sulfate salt, chloride salt, carbonate salt, phosphate salt or hydroxide.

10. A method for preparing a composition for delivering virus according to Claim 1, comprising:
(a) a step of dissolving virus in aqueous solvent;
(b) a step of dissolving each of amphiphilic block copolymer and salt of polylactic acid in organic solvent; and
(c) a step of mixing the solution of the steps (a) and (b) to form an emulsion,
wherein the amphiphilic block copolymer is an A-B type block copolymer comprising a hydrophilic A block and a hydrophobic B block, wherein the hydrophilic A block is one or more selected from the group consisting of monomethoxy polyethylene glycol, monoacetoxy polyethylene glycol, polyethylene glycol, a copolymer of polyethylene and propylene glycol, and polyvinyl pyrrolidone, and the hydrophobic B block is one or more selected from the group consisting of polyester, polyanhydride, polyamino acid, polyorthoester and polyphosphazine; and
wherein the salt of polylactic acid is one or more selected from the group consisting of the compounds of the following Formulas 1 to 6:
[Formula 1] **RO-CHZ-[A]ₙ-[B]ₘ-COOM**
wherein A is -COO-CHZ-; B is -COO-CHY-, -COO-CH₂CH₂CH₂CH₂CH₂- or -COO-CH₂CH₂OCH₂-; R is a hydrogen atom, or acetyl, benzoyl, decanoyl, palmitoyl, methyl or ethyl; each of Z and Y is a hydrogen atom, or methyl or phenyl; M is Na, K or Li; n is an integer of from 1 to 30; and m is an integer of from 0 to 20;
[Formula 2] **RO-CHZ-[COO-CHX]ₚ-[COO-CHY']_{q}-COO-CHZ-COOM**
wherein X is methyl; Y' is a hydrogen atom or phenyl; p is an integer of from 0 to 25, q is an integer of from 0 to 25, with the proviso that p + q is an integer of from 5 to 25; R is a hydrogen atom, or acetyl, benzoyl, decanoyl, palmitoyl, methyl or ethyl; M is Na, K or
Li; and Z is a hydrogen atom, methyl or phenyl.;
[Formula 3] **RO-PAD-COO-W-M'**
wherein W-M' is 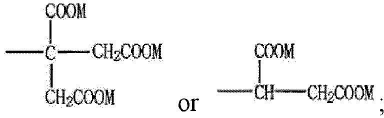 PAD is selected from the group consisting of D,L-polylactic acid, D-polylactic acid, polymandelic acid, copolymer of D,L-lactic acid and glycolic acid, copolymer of D,L-lactic acid and mandelic acid, copolymer of D,L-lactic acid and caprolactone, and copolymer of D,L-lactic acid and 1,4-dioxane-2-one; R is a hydrogen atom, or acetyl, benzoyl, decanoyl, palmitoyl, methyl or ethyl; and M is independently Na, K or Li;
[Formula 4] **S-O-PAD-COO-Q**
wherein S is 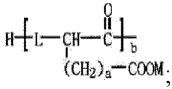 L is -NR₁- or -O-, wherein R₁ is a hydrogen atom or C₁₋₁₀ alkyl; Q is -CH₃, -CH₂CH₃, -CH₂CH₂CH₃, -CH₂CH₂CH₂CH₃, or -CH₂C₆H₅; a is an integer of from 0 to 4; b is an integer of from 1 to 10; M is Na, K or Li; and PAD is one or more selected from the group consisting of D,L-polylactic acid, D-polylactic acid, polymandelic acid, copolymer of D,L-lactic acid and glycolic acid, copolymer of D,L-lactic acid and mandelic acid, copolymer of D,L-lactic acid and caprolactone, and copolymer of D,L-lactic acid and 1,4-dioxane-2-one; 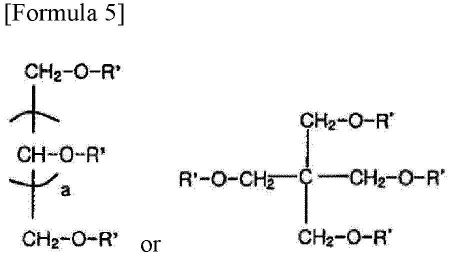
wherein R' is -PAD-O-C(O)-CH₂CH₂-C(O)-OM, wherein PAD is selected from the group consisting of D,L-polylactic acid, D-polylactic acid, polymandelic acid, copolymer of D,L-lactic acid and glycolic acid, copolymer of D,L-lactic acid and mandelic acid, copolymer of D,L-lactic acid and caprolactone, and copolymer of D,L-lactic acid and 1,4-dioxane-2-one, M is Na, K or Li; and a is an integer of from 1 to 4;
[Formula 6] **YO-[-C(O)-(CHX)ₐ-O-]ₘ-C(O)-R-C(O)-[-O-(CHX')_{b}-C(O)-]ₙ-OZ**
wherein X and X' are independently hydrogen, C₁₋₁₀ alkyl or C₆₋₂₀ aryl; Y and Z are independently Na, K or Li; m and n are independently an integer of from 0 to 95, with the proviso that 5 < m + n < 100; a and b are independently an integer of from 1 to 6; and R is -(CH₂)ₖ-, C₂₋₁₀ divalent alkenyl, C₆₋₂₀ divalent aryl or a combination thereof, wherein k is an integer of from 0 to 10.

11. The method for preparing a composition for delivering virus of Claim 10, wherein step (b) further comprises a step of dissolving cationic compound in organic solvent.

12. The method for preparing a composition for delivering virus of Claim 10, further comprising (d) a step of selectively removing the organic solvent from the emulsion obtained in step (c).

13. The method for preparing a composition for delivering virus of Claim 12, further comprising (e) a step of adding divalent or trivalent metal ion after step (d).

## Patentansprüche

1. Zusammensetzung zur Virusabgabe, umfassend:
ein Virus als wirksamen Bestandteil, ein amphiphiles Blockcopolymer und ein Salz der Polymilchsäure,
wobei das Virus in einer Nanopartikelstruktur eingeschlossen ist, die durch das amphiphile Blockcopolymer und das Salz der Polymilchsäure ausgeformt ist,
wobei das amphiphile Blockcopolymer ein Blockcopolymer vom A-B-Typ ist, das einen hydrophilen A-Block und einen hydrophoben B-Block umfasst, wobei der hydrophile A-Block einer oder mehrere ist bzw. sind, die aus der Gruppe ausgewählt sind, die aus Monomethoxypolyethylenglykol, Monoacetoxypolyethylenglykol, Polyethylenglykol, einem Copolymer von Polyethylen und Propylenglykol, sowie Polyvinylpyrrolidon besteht, und der hydrophobe B-Block einer oder mehrere ist bzw. sind, die aus der Gruppe ausgewählt sind, die aus Polyester, Polyanhydrid, Polyaminosäure, Polyorthoester und Polyphosphazin besteht, und
wobei das Salz der Polymilchsäure eines oder mehrere ist bzw. sind, die aus der Gruppe ausgewählt sind, die aus den Verbindungen der folgenden Formeln 1 bis 6 besteht:
[Formel 1] **RO-CHZ-[A]ₙ-[B]ₘ-COOM**
wobei A für -COO-CHZ- steht, B für -COO-CHY-, -COO-CH₂CH₂CH₂CH₂CH₂- oder -COO-CH₂CH₂OCH₂- steht, R für ein Wasserstoffatom oder Acetyl, Benzoyl, Decanoyl, Palmitoyl, Methyl oder Ethyl steht, Z und Y jeweils für ein Wasserstoffatom oder Methyl oder Phenyl stehen, M für Na, K oder Li steht, n für eine ganze Zahl von 1 bis 30 steht und m für eine ganze Zahl von 0 bis 20 steht;
[Formel 2] **RO-CHZ-[COO-CHX]ₚ-[COO-CHY']_{q}-COO-CHZ-COOM**
wobei X für Methyl steht; Y' für ein Wasserstoffatom oder Phenyl steht, p für eine ganze Zahl von 0 bis 25 steht, q für eine ganze Zahl von 0 bis 25 steht, mit der Maßgabe, dass p + q eine ganze Zahl von 5 bis 25 ist, R für ein Wasserstoffatom oder Acetyl, Benzoyl, Decanoyl, Palmitoyl, Methyl oder Ethyl steht, M für Na, K oder Li steht und Z für ein Wasserstoffatom, Methyl oder Phenyl steht;
[Formel 3] **RO-PAD-COO-W-M'**
wobei W-M' für 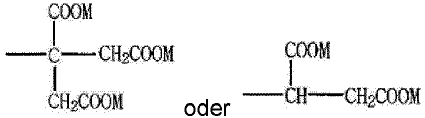 steht, PAD aus der Gruppe ausgewählt ist, die aus D,L-Polymilchsäure, D-Polymilchsäure, Polymandelsäure, Copolymer von D,L-Milchsäure und Glykolsäure, Copolymer von D,L-Milchsäure und Mandelsäure, Copolymer von D,L-Milchsäure und Caprolacton sowie Copolymer von D,L-Milchsäure und 1,4-Dioxan-2-on besteht, R für ein Wasserstoffatom oder Acetyl, Benzoyl, Decanoyl, Palmitoyl, Methyl oder Ethyl steht und M unabhängig für Na, K oder Li steht;
[Formel 4] **S-O-PAD-COO-Q**
wobei S für 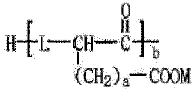 steht, L für -NR₁- oder -O- steht, wobei R₁ für ein Wasserstoffatom oder C₁₋₁₀-Alkyl steht, Q für -CH₃, -CH₂CH₃, -CH₂CH₂CH₃, -CH₂CH₂CH₂CH₃ oder -CH₂C₆H₅ steht, a für eine ganze Zahl von 0 bis 4 steht, b für eine ganze Zahl von 1 bis 10 steht, M für Na, K oder Li steht und PAD eines oder mehrere ist bzw. sind, die aus der Gruppe ausgewählt sind, die aus D,L-Polymilchsäure, D-Polymilchsäure, Polymandelsäure, Copolymer von D,L-Milchsäure und Glykolsäure, Copolymer von D,L-Milchsäure und Mandelsäure, Copolymer von D,L-Milchsäure und Caprolacton sowie Copolymer von D,L-Milchsäure und 1,4-Dioxan-2-on besteht; 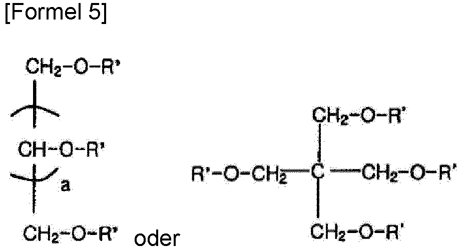
wobei R' für -PAD-O-C(O)-CH₂CH₂-C(O)-OM steht, wobei PAD aus der Gruppe ausgewählt ist, die aus D,L-Polymilchsäure, D-Polymilchsäure, Polymandelsäure, Copolymer von D,L-Milchsäure und Glykolsäure, Copolymer von D,L-Milchsäure und Mandelsäure, Copolymer von D,L-Milchsäure und Caprolacton sowie Copolymer von D,L-Milchsäure und 1,4-Dioxan-2-on besteht, M für Na, K oder Li steht und a für eine ganze Zahl von 1 bis 4 steht;
[Formel 6] **YO-[-C(O)-(CHX)ₐ-O-]ₘ-C(O)-R-C(O)-[-O-(CHX')_{b}-C(O)-]ₙ-OZ**
wobei X und X' unabhängig voneinander für Wasserstoff, C₁₋₁₀-Alkyl oder C₆₋₂₀-Aryl stehen, Y und Z unabhängig voneinander für Na, K oder Li stehen, m und n unabhängig voneinander für eine ganze Zahl von 0 bis 95 stehen, mit der Maßgabe, dass 5 < m + n < 100 ist, a und b unabhängig voneinander für eine ganze Zahl von 1 bis 6 stehen und R für -(CH₂)ₖ-, zweiwertiges C₂₋₁₀₋Alkenyl, zweiwertiges C₆₋₂₀-Aryl oder eine Kombination davon steht, wobei k für eine ganze Zahl von 0 bis 10 steht.

2. Zusammensetzung zur Virusabgabe gemäß Anspruch 1, wobei das Virus ein onkolytisches Virus ist.

3. Zusammensetzung zur Virusabgabe gemäß Anspruch 2, wobei das onkolytische Virus eines oder mehrere ist bzw. sind, die aus der Gruppe ausgewählt sind, die aus Adenovirus, Vacciniavirus, Herpes-simplex-Virus (HSV) und vesikulärem Stomatitisvirus (VSV) besteht.

4. Zusammensetzung zur Virusabgabe gemäß Anspruch 1, wobei eine Hydroxylgruppe am Ende des hydrophoben B-Blocks durch eines oder mehrere, das bzw. die aus der Gruppe ausgewählt sind, die aus Tocopherol, Cholesterin und C₁₀₋₂₄-Fettsäure besteht, modifiziert ist.

5. Zusammensetzung zur Virusabgabe gemäß Anspruch 1, wobei das Salz der Polymilchsäure eine Verbindung der Formel 1 oder 2 ist.

6. Zusammensetzung zur Virusabgabe gemäß Anspruch 1, die des Weiteren eine kationische Verbindung umfasst.

7. Zusammensetzung zur Virusabgabe gemäß Anspruch 1, die des Weiteren ein zweiwertiges oder dreiwertiges Metallion umfasst.

8. Zusammensetzung zur Virusabgabe gemäß Anspruch 7, wobei das zweiwertige oder dreiwertige Metallion eines oder mehrere ist bzw. sind, die aus der Gruppe ausgewählt sind, die aus Calcium (Ca²⁺), Magnesium (Mg²⁺), Barium (Ba²⁺), Chrom (Cr³⁺), Eisen (Fe³⁺), Mangan (Mn²⁺), Nickel (Ni²⁺), Kupfer (Cu²⁺), Zink (Zn²⁺) und Aluminium (Al³⁺) besteht.

9. Zusammensetzung zur Virusabgabe gemäß Anspruch 7, wobei das zweiwertige oder dreiwertige Metallion in Form eines Sulfatsalzes, Chloridsalzes, Carbonatsalzes, Phosphatsalzes oder Hydroxids enthalten ist.

10. Verfahren zur Herstellung einer Zusammensetzung zur Virusabgabe gemäß Anspruch 1, wobei das Verfahren folgende Schritte umfasst:
(a) einen Schritt des Lösens des Virus in wässrigem Lösemittel,
(b) einen Schritt des Lösens von jeweils einem amphiphilen Blockcopolymer und einem Salz der Polymilchsäure in einem organischen Lösungsmittel, und
(c) einen Schritt des Mischens der Lösung der Schritte (a) und (b), um eine Emulsion zu bilden,
wobei das amphiphile Blockcopolymer ein Blockcopolymer vom A-B-Typ ist, das einen hydrophilen A-Block und einen hydrophoben B-Block umfasst, wobei der hydrophile A-Block einer oder mehrere ist bzw. sind, die aus der Gruppe ausgewählt sind, die aus Monomethoxypolyethylenglykol, Monoacetoxy-polyethylenglykol, Polyethylenglykol, einem Copolymer von Polyethylen und Propylenglykol, sowie Polyvinylpyrrolidon besteht, und der hydrophobe B-Block einer oder mehrere ist bzw. sind, die aus der Gruppe ausgewählt sind, die aus Polyester, Polyanhydrid, Polyaminosäure, Polyorthoester und Polyphosphazin besteht, und
wobei das Salz der Polymilchsäure eines oder mehrere ist bzw. sind, die aus der Gruppe ausgewählt sind, die aus den Verbindungen der folgenden Formeln 1 bis 6 besteht:
[Formel 1] **RO-CHZ-[A]ₙ-[B]ₘ-COOM**
wobei A für -COO-CHZ- steht, B für -COO-CHY-, -COO-CH₂CH₂CH₂CH₂CH₂- oder -COO-CH₂CH₂OCH₂- steht, R für ein Wasserstoffatom oder Acetyl, Benzoyl, Decanoyl, Palmitoyl, Methyl oder Ethyl steht, Z und Y jeweils für ein Wasserstoffatom oder Methyl oder Phenyl stehen, M für Na, K oder Li steht, n für eine ganze Zahl von 1 bis 30 steht und m für eine ganze Zahl von 0 bis 20 steht;
[Formel 2] **RO-CHZ-[COO-CHX]ₚ-[COO-CHY']_{q}-COO-CHZ-COOM**
wobei X für Methyl steht; Y' für ein Wasserstoffatom oder Phenyl steht, p für eine ganze Zahl von 0 bis 25 steht, q für eine ganze Zahl von 0 bis 25 steht, mit der Maßgabe, dass p + q eine ganze Zahl von 5 bis 25 ist, R für ein Wasserstoffatom oder Acetyl, Benzoyl, Decanoyl, Palmitoyl, Methyl oder Ethyl steht, M für Na, K oder Li steht und Z für ein Wasserstoffatom, Methyl oder Phenyl steht;
[Formel 3] **RO-PAD-COO-W-M'**
wobei W-M' für 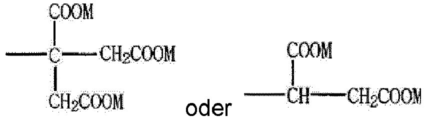 steht, PAD aus der Gruppe ausgewählt ist, die aus D,L-Polymilchsäure, D-Polymilchsäure, Polymandelsäure, Copolymer von D,L-Milchsäure und Glykolsäure, Copolymer von D,L-Milchsäure und Mandelsäure, Copolymer von D,L-Milchsäure und Caprolacton sowie Copolymer von D,L-Milchsäure und 1,4-Dioxan-2-on besteht, R für ein Wasserstoffatom oder Acetyl, Benzoyl, Decanoyl, Palmitoyl, Methyl oder Ethyl steht und M unabhängig für Na, K oder Li steht;
[Formel 4] **S-O-PAD-COO-Q**
wobei S für 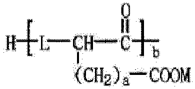 steht, L für -NR₁- oder -O- steht, wobei R₁ für ein Wasserstoffatom oder C₁₋₁₀-Alkyl steht, Q für -CH₃, -CH₂CH₃, -CH₂CH₂CH₃, -CH₂CH₂CH₂CH₃ oder -CH₂C₆H₅ steht, a für eine ganze Zahl von 0 bis 4 steht, b für eine ganze Zahl von 1 bis 10 steht, M für Na, K oder Li steht und PAD eines oder mehrere ist bzw. sind, die aus der Gruppe ausgewählt sind, die aus D,L-Polymilchsäure, D-Polymilchsäure, Polymandelsäure, Copolymer von D,L-Milchsäure und Glykolsäure, Copolymer von D,L-Milchsäure und Mandelsäure, Copolymer von D,L-Milchsäure und Caprolacton sowie Copolymer von D,L-Milchsäure und 1,4-Dioxan-2-on besteht; 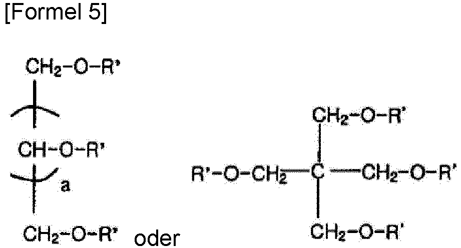
wobei R' für -PAD-O-C(O)-CH₂CH₂-C(O)-OM steht, wobei PAD aus der Gruppe ausgewählt ist, die aus D,L-Polymilchsäure, D-Polymilchsäure, Polymandelsäure, Copolymer von D,L-Milchsäure und Glykolsäure, Copolymer von D,L-Milchsäure und Mandelsäure, Copolymer von D,L-Milchsäure und Caprolacton sowie Copolymer von D,L-Milchsäure und 1,4-Dioxan-2-on besteht, M für Na, K oder Li steht und a für eine ganze Zahl von 1 bis 4 steht;
[Formel 6] **YO-[-C(O)-(CHX)ₐ-O-]ₘ-C(O)-R-C(O)-[-O-(CHX')_{b}-C(O)-]ₙ-OZ**
wobei X und X' unabhängig voneinander für Wasserstoff, C₁₋₁₀-Alkyl oder C₆₋₂₀-Aryl stehen, Y und Z unabhängig voneinander für Na, K oder Li stehen, m und n unabhängig voneinander für eine ganze Zahl von 0 bis 95 stehen, mit der Maßgabe, dass 5 < m + n < 100 ist, a und b unabhängig voneinander für eine ganze Zahl von 1 bis 6 stehen und R für -(CH₂)ₖ-, zweiwertiges C₂₋₁₀₋Alkenyl, zweiwertiges C₆₋₂₀-Aryl oder eine Kombination davon steht, wobei k für eine ganze Zahl von 0 bis 10 steht.

11. Verfahren zur Herstellung einer Zusammensetzung zur Virusabgabe gemäß Anspruch 10, wobei Schritt (b) des Weiteren einen Schritt des Lösens einer kationischen Verbindung in einem organischen Lösemittel umfasst.

12. Verfahren zur Herstellung einer Zusammensetzung zur Virusabgabe gemäß Anspruch 10, das des Weiteren (d) einen Schritt des selektiven Entfernens des organischen Lösemittels aus der Emulsion, die in Schritt (c) erhalten wurde, umfasst.

13. Verfahren zur Herstellung einer Zusammensetzung zur Virusabgabe gemäß Anspruch 12, das des Weiteren (e) einen Schritt des Zugebens eines zweiwertigen oder dreiwertigen Metallions nach Schritt (d) umfasst.

## Revendications

1. Composition pour livrer un virus, comprenant : un virus en tant que principe actif ; un copolymère séquencé amphiphile ; et un sel d'acide polylactique ;
dans laquelle le virus est piégé dans une structure nanoparticulaire formée par le copolymère séquencé amphiphile et le sel d'acide polylactique dans laquelle le copolymère séquencé amphiphile est un copolymère séquencé de type A-B comprenant une séquence hydrophile A et une séquence hydrophobe B, dans laquelle la séquence hydrophile A est une ou plusieurs sélectionnées dans le groupe constitué de monométhoxy polyéthylène glycol, de monoacétoxy polyéthylène glycol, de polyéthylène glycol, d'un copolymère de polyéthylène et de propylène glycol, et de polyvinyl pyrrolidone, et la séquence hydrophobe B est une ou plusieurs sélectionnées dans le groupe constitué de polyester, de polyanhydride, d'un polyamino acide, de polyorthoester et de polyphosphazine ; et
dans laquelle le sel d'acide polylactique est un ou plusieurs sélectionnés dans le groupe constitué des composés des formules 1 à 6 suivantes :
[formule 1] **RO-CHZ-[A]ₙ-[B]ₘ-COOM**
dans laquelle A est -COO-CHZ- ; B est -COO-CHY-, -COO-CH₂CH₂CH₂CH₂CH₂- ou -COO-CH₂CH₂OCH₂- ; R est un atome d'hydrogène, ou un acétyle, un benzoyle, un décanoyle, un palmitoyle, un méthyle ou un éthyle ; chacun de Z et Y est un atome d'hydrogène, ou un méthyle ou un phényle ; M est Na, K ou Li ; n est un nombre entier de 1 à 30 ; et m est un nombre entier de 0 à 20 ;
[formule 2] **RO-CHZ-[COO-CHX]ₚ-[COO-CHY']_{q}-COO-CHZ-COOM**
dans laquelle X est un méthyle ; Y' est un atome d'hydrogène ou un phényle ; p est un nombre entier de 0 à 25, q est un nombre entier de 0 à 25, à condition que p + q soit un nombre entier de 5 à 25 ; R est un atome d'hydrogène, ou un acétyle, un benzoyle, un décanoyle, un palmitoyle, un méthyle ou un éthyle ; M est Na, K ou Li ; et Z est un atome d'hydrogène, un méthyle ou un phényle ;
[formule 3] **RO-PAD-COO-W-M'**
dans laquelle W-M' est 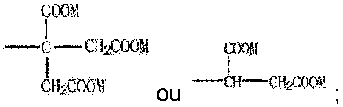 PAD est sélectionné dans le groupe constitué de l'acide D,L-polylactique, de l'acide D-polylactique, de l'acide polymandélique, d'un copolymère d'acide D,L-lactique et d'acide glycolique, d'un copolymère d'acide D,L-lactique et d'acide mandélique, d'un copolymère d'acide D,L-lactique et de caprolactone, et d'un copolymère d'acide D,L-lactique et de 1,4-dioxane-2-one ; R est un atome d'hydrogène, ou un acétyle, un benzoyle, un décanoyle, un palmitoyle, un méthyle ou un éthyle ; et M est indépendamment Na, Kou Li ;
[formule 4] **S-O-PAD-COO-Q**
dans laquelle S est 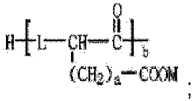 L est -NR₁- ou -O-, dans laquelle R₁ est un atome d'hydrogène ou un alkyle en C₁₋₁₀ ; Q est -CH₃, -CH₂CH₃, -CH₂CH₂CH₃, -CH₂CH₂CH₂CH₃, ou -CH₂C₆H₅ ; a est un nombre entier de 0 à 4 ; b est un nombre entier de 1 à 10 ; M est Na, K ou Li ; et PAD est un ou plusieurs sélectionnés dans le groupe constitué de l'acide D,L-polylactique, de l'acide D-polylactique, de l'acide polymandélique, d'un copolymère d'acide D,L-lactique et d'acide glycolique, d'un copolymère d'acide D,L-lactique et d'acide mandélique, d'un copolymère d'acide D,L-lactique et de caprolactone, et d'un copolymère d'acide D,L-lactique et de 1,4-dioxane-2-one ; 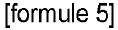 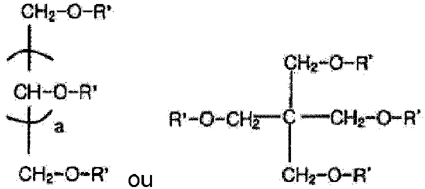
dans laquelle R' est -PAD-O-C(O)-CH₂CH₂-C(O)-OM, dans laquelle PAD est sélectionné dans le groupe constitué de l'acide D,L-polylactique, de l'acide D-polylactique, de l'acide polymandélique, d'un copolymère d'acide D,L-lactique et d'acide glycolique, d'un copolymère d'acide D,L-lactique et d'acide mandélique, d'un copolymère d'acide D,L-lactique et de caprolactone, et d'un copolymère d'acide D,L-lactique et de 1,4-dioxane-2-one, M est Na, K ou Li ; et a est un nombre entier de 1 à 4 ;
[formule 6] **YO-[-C(O)-(CHX)ₐ-O-]ₘ-C(O)-R-C(O)-[-O-(CHX')_{b}-C(O)-]ₙ-OZ**
dans laquelle X et X' sont indépendamment un hydrogène, un alkyle en C₁₋₁₀ ou un aryle en C₆₋₂₀ ; Y et Z sont indépendamment Na, K ou Li ; m et n sont indépendamment un nombre entier de 0 à 95, à condition que 5 < m + n < 100 ; a et b sont indépendamment un nombre entier de 1 à 6 ; et R est -(CH₂)ₖ-, un alcényle divalent en C₂₋₁₀, un aryle divalent en C₆₋₂₀ ou une combinaison de ceux-ci, dans laquelle k est un nombre entier de 0 à 10.

2. Composition pour livrer un virus selon la revendication 1, dans laquelle le virus est un virus oncolytique.

3. Composition pour livrer un virus selon la revendication 2, dans laquelle le virus oncolytique est un ou plusieurs sélectionnés dans le groupe constitué d'un adénovirus, d'un virus de la vaccine, d'un virus herpes simplex (HSV) et d'un virus de la stomatite vésiculaire (VSV).

4. Composition pour livrer un virus selon la revendication 1, dans laquelle un groupe hydroxyle à la fin de la séquence hydrophobe B est modifié par un ou plusieurs sélectionnés dans le groupe constitué du tocophérol, du cholestérol, et d'un acide gras en C₁₀₋₂₄.

5. Composition pour livrer un virus selon la revendication 1, dans laquelle le sel de l'acide polylactique est un composé de formule 1 ou 2.

6. Composition pour livrer un virus selon la revendication 1, comprenant en outre un composé cationique.

7. Composition pour livrer un virus selon la revendication 1, comprenant en outre un ion métallique divalent ou trivalent.

8. Composition pour livrer un virus selon la revendication 7, dans laquelle l'ion métallique divalent ou trivalent est un ou plusieurs sélectionnés dans le groupe constitué du calcium (Ca²⁺), du magnésium (Mg²⁺), du baryum (Ba²⁺), du chrome (Cr³⁺), du fer (Fe³⁺), du manganèse (Mn²⁺), du nickel (Ni²⁺), du cuivre (Cu²⁺), du zinc (Zn²⁺) et de l'aluminium (Al³⁺).

9. Composition pour livrer un virus selon la revendication 7, dans laquelle l'ion métallique divalent ou trivalent est compris sous la forme d'un sel de sulfate, d'un sel de chlorure, d'un sel de carbonate, d'un sel de phosphate ou d'un hydroxyde.

10. Procédé de préparation d'une composition pour livrer un virus selon la revendication 1, comprenant :
(a) une étape de dissolution d'un virus dans un solvant aqueux ;
(b) une étape de dissolution de chacun du polymère séquencé amphiphile et du sel d'acide polylactique dans un solvant organique ; et
(c) une étape de mélange de la solution des étapes (a) et (b) pour former une émulsion,
dans lequel le copolymère séquencé amphiphile est un copolymère séquencé de type A-B comprenant une séquence hydrophile A et une séquence hydrophobe B, dans lequel la séquence hydrophile A est une ou plusieurs sélectionnées dans le groupe constitué de monométhoxy polyéthylène glycol, de monoacétoxy polyéthylène glycol, de polyéthylène glycol, d'un copolymère de polyéthylène et de propylène glycol, et de polyvinyl pyrrolidone, et la séquence hydrophobe B est une ou plusieurs sélectionnées dans le groupe constitué de polyester, de polyanhydride, d'un polyamino acide, de polyorthoester et de polyphosphazine ; et
dans lequel le sel de l'acide polylactique est un ou plusieurs sélectionnés dans le groupe constitué des composés des formules 1 à 6 suivantes :
[formule 1] **RO-CHZ-[A]ₙ-[B]ₘ-COOM**
dans laquelle A est -COO-CHZ- ; B est -COO-CHY-, -COO-CH₂CH₂CH₂CH₂CH₂- ou -COO-CH₂CH₂OCH₂- ; R est un atome d'hydrogène, ou un acétyle, un benzoyle, un décanoyle, un palmitoyle, un méthyle ou un éthyle ; chacun de Z et Y est un atome d'hydrogène, ou un méthyle ou un phényle ; M est Na, K ou Li ; n est un nombre entier de 1 à 30 ; et m est un nombre entier de 0 à 20 ;
[Formule 2] **RO-CHZ-[COO-CHX]ₚ-[COO-CHY']_{q}-COO-CHZ-COOM**
dans laquelle X est un méthyle ; Y' est un atome d'hydrogène ou un phényle ; p est un nombre entier de 0 à 25, q est un nombre entier de 0 à 25, à condition que p + q soit un nombre entier de 5 à 25 ; R est un atome d'hydrogène, ou un acétyle, un benzoyle, un décanoyle, un palmitoyle, un méthyle ou un éthyle ; M est Na, K ou Li ; et Z est un atome d'hydrogène, un méthyle ou un phényle ;
[formule 3] **RO-PAD-COO-W-M'**
dans laquelle W-M' est 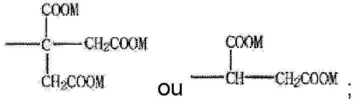 PAD est sélectionné dans le groupe constitué de l'acide D,L-polylactique, de l'acide D-polylactique, de l'acide polymandélique, d'un copolymère d'acide D,L-lactique et d'acide glycolique, d'un copolymère d'acide D,L-lactique et d'acide mandélique, d'un copolymère d'acide D,L-lactique et de caprolactone, et d'un copolymère d'acide D,L-lactique et de 1,4-dioxane-2-one ; R est un atome d'hydrogène, ou un acétyle, un benzoyle, un décanoyle, un palmitoyle, un méthyle ou un éthyle ; et M est indépendamment Na, Kou Li ;
[formule 4] **S-O-PAD-COO-Q**
dans laquelle S est 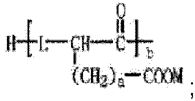 L est -NR₁- ou -O-, dans laquelle R₁ est un atome d'hydrogène ou un alkyle en C₁₋₁₀ ; Q est -CH₃, -CH₂CH₃, - CH₂CH₂CH₃, -CH₂CH₂CH₂CH₃, ou -CH₂C₆H₅ ; a est un nombre entier de 0 à 4 ; b est un nombre entier de 1 à 10 ; M est Na, K ou Li ; et PAD est un ou plusieurs sélectionnés dans le groupe constitué de l'acide D,L-polylactique, de l'acide D-polylactique, de l'acide polymandélique, d'un copolymère d'acide D,L-lactique et d'acide glycolique, d'un copolymère d'acide D,L-lactique et d'acide mandélique, d'un copolymère d'acide D,L-lactique et de caprolactone, et d'un copolymère d'acide D,L-lactique et de 1,4-dioxane-2-one ; 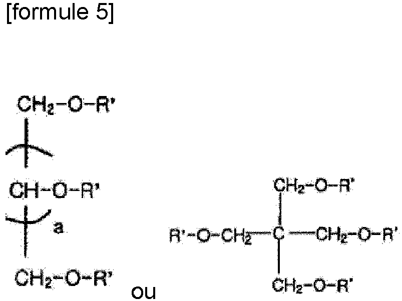
dans laquelle R' est -PAD-O-C(O)-CH₂CH₂-C(O)-OM, dans laquelle PAD est sélectionné dans le groupe constitué de l'acide D,L-polylactique, de l'acide D-polylactique, de l'acide polymandélique, d'un copolymère d'acide D,L-lactique et d'acide glycolique, d'un copolymère d'acide D,L-lactique et d'acide mandélique, d'un copolymère d'acide D,L-lactique et de caprolactone, et d'un copolymère d'acide D,L-lactique et de 1,4-dioxane-2-one, M est Na, K ou Li ; et a est un nombre entier de 1 à 4 ;
[formule 6] **YO-[-C(O)-(CHX)ₐ-O-]ₘ-C(O)-R-C(O)-[-O-(CHX')_{b}-C(O)-]ₙ-OZ**
dans laquelle X et X' sont indépendamment un hydrogène, un alkyle en C₁₋₁₀ ou un aryle en C₆₋₂₀ ; Y et Z sont indépendamment Na, K ou Li ; m et n sont indépendamment un nombre entier de 0 à 95, à condition que 5 < m + n < 100 ; a et b sont indépendamment un nombre entier de 1 à 6 ; et R est -(CH₂)ₖ-, un alcényle divalent en C₂₋₁₀, un aryle divalent en C₆₋₂₀ ou une combinaison de ceux-ci, dans laquelle k est un nombre entier de 0 à 10.

11. Procédé de préparation d'une composition pour livrer un virus selon la revendication 10, dans lequel l'étape (b) comprend en outre une étape de dissolution d'un composé cationique dans un solvant organique.

12. Procédé de préparation d'une composition pour livrer un virus selon la revendication 10, comprenant en outre (d) une étape de retrait sélectif du solvant organique de l'émulsion obtenue à l'étape (c).

13. Procédé de préparation d'une composition pour livrer un virus selon la revendication 12, comprenant en outre (e) une étape d'ajout d'un ion métallique divalent ou trivalent après l'étape (d).
